# EUROPÄISCHE PATENTSCHRIFT

(11) **EP 3 160 483 B1**
(45) Veröffentlichungstag und Bekanntmachung des Hinweises auf die Patenterteilung: **28.02.2018**
(21) Anmeldenummer: 15741130.7
(22) Anmeldetag: 26.06.2015
(51) Int. Cl.: A61K 36/28, A61P 1/16

(54) **MARIENDISTELEXTRAKT AUS MARIENDISTELFRÜCHTESCHALEN, VERFAHREN ZUR HERSTELLUNG UND VERWENDUNG**
MILK THISTLE EXTRACT OF FRUIT SHELLS OF SILYBUM MARIANUM, PROCESS OF MANUFACTURE AND USE
EXTRAIT DE SILYBUM MARIANUM À PARTIR DES ÉPLUCHURES DE FRUITS DE SILYBUM MARIANUM, PROCÉDÉ DE MANUFACTURE ET USAGE

(30) Priorität: 26.06.2014 EP 14174384
(43) Veröffentlichungstag der Anmeldung: 03.05.2017
(73) Patentinhaber: Bionorica SE, 92318 Neumarkt (DE)
(72) Erfinder: INTELMANN, Daniel, 6020 Innsbruck (AT); KARLSEDER, Alban, 6063 Rum (AT)
(74) Vertreter: Simandi, Claus
(86) Internationale Anmeldenummer: PCT/EP2015/064600
(87) Internationale Veröffentlichungsnummer: WO 2015/197854

(56) Entgegenhaltungen:
- EP-A1- 2 020 238
- DATABASE WPI Week 201414 Thomson Scientific, London, GB; AN 2014-B83467 XP002728650, & CN 103 408 539 A (TIANJIN TAIYANG PHARM CO LTD) 27. November 2013 (2013-11-27)
- DATABASE WPI Week 201223 Thomson Scientific, London, GB; AN 2011-M48048 XP002728651, & CN 102 174 041 A (JIANGSU ZHONGXING PHARM CO LTD) 7. September 2011 (2011-09-07)
- DATABASE WPI Week 201210 Thomson Scientific, London, GB; AN 2011-H83903 XP002728652, & CN 102 079 745 A (BAI X) 1. Juni 2011 (2011-06-01)
- DATABASE WPI Week 199612 Thomson Scientific, London, GB; AN 1996-114539 XP002728653, & RU 2 038 091 C1 (KHARK ZDOROVE CHEM PHARM PRODN ASSOC) 27. Juni 1995 (1995-06-27)
- DATABASE WPI Week 201228 Thomson Scientific, London, GB; AN 2011-P34180 XP002728654, & CN 102 219 781 A (DI F) 19. Oktober 2011 (2011-10-19) in der Anmeldung erwähnt
- DATABASE EMBASE [Online] ELSEVIER SCIENCE PUBLISHERS, AMSTERDAM, NL; 2007, DANOS B: "Formation of flavanolignane in the white-flowered variety (Szibilla(R)) of Silybum marianum in relation to fruit development", XP002728663, Database accession no. EMB-2007194354 & ACTA PHARMACEUTICA HUNGARICA 2007 HU, Bd. 77, Nr. 1, 2007, Seiten 47-51, ISSN: 0001-6659

## Beschreibung

Die vorliegende Erfindung betrifft ein Verfahren zur Herstellung eines Mariendistelextrakts aus Mariendistelfrüchteschalen und dessen Verwendung, insbesondere zur Therapie und Prophylaxe von Lebererkrankungen und Krebs-und Tumorerkrankungen.

Die Mariendistel (Milk thistle, Silybum marianum oder Carduus marianus) ist eine besonders in Südwest- und Mitteleuropa verbreitete kultivierte Pflanze und in Eurasien, Nordamerika und Südamerika naturalisiert. Weitere Anbaugebiete befinden sich in China.

Die Wirksamkeit der Droge zur Prophylaxe und Behandlung von verschiedenen Formen der Leber- und Gallendysfunktionen als auch von Krebs- und Tumorerkrankungen ist bekannt. Die Droge besteht aus den reifen, vom Pappus befreiten Früchten mit einem Mindestgehalt von 1,5 % Silymarin (Pharmacopoea Europaea (Ph. Eur. 6.0/1860), europäisches Arzneibuch 2007). Bereits aus dem Altertum sind Tinkturen (zumeist alkoholisch-wässrige Auszüge aus der Droge) aus Mariendistel bekannt. Insbesondere isoliertes Silymarin ist besonders bedeutend und geeignet (z.B. DE 1 923 082 (Madaus)). Mariendistelextrakte sind gängige Arzneimittel und ebenfalls Bestandteil von Kombinationspräparaten, wie das Phytopharmakon Iberogast®. Die Anmelderin selbst vertreibt z.B. das Präparat Silimarit® auf der Basis eines standardisierten Mariendistelfrüchte-Trockenextraktes in Form von Weichkapseln. Der Hersteller Finzelberg GmbH & Co. KG stellt beispielsweise solche Mariendistelfrüchte-Trockenextrakte in unterschiedlichen Arzneiformen her. Ein bekanntes MariendistelfrüchteTrockenextrakt-Produkt ist z.B. Legalon forte®.

Silymarin ist ein Flavonolignan-Komplex bzw. Polyhydroxyphenylchromanonen und wurde erstmals aus der Pflanze in den 1960 -iger Jahren isoliert (Dissertation Janiak Bernhard, Juni 1960, FU-Berlin (DE 2020407), Pelter A., Hänsel R., Tetrahedron Letters, 25, (1968)).

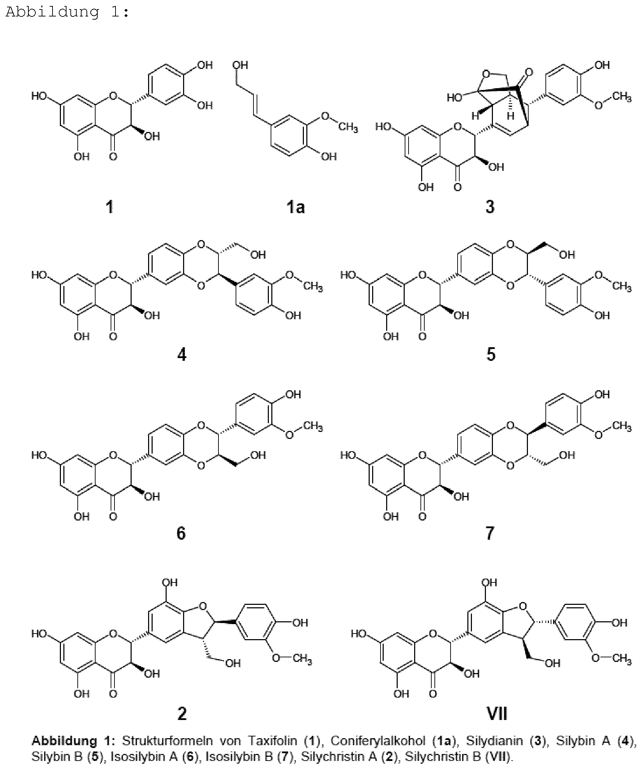

Silymarin besteht aus einem isobaren Gemisch von Flavonolignanen mit den Hauptbestandteilen Silybin A und B (Silibinin A und B), Isosilybin A und B (Isosilibinin A und B), Silydianin (Silidianin) und Silychristin (Silichristin). Bei diesen Flavonolignanen ist das Taxifolin (1) mit Coniferyl-Alkohol (1a) verknüpft, siehe Abbildung 1 (Vgl. Kim, N.-C.; Graf, T. N.; Sparacino, C. M.; Wani, M. C.; Wall, M. E., Complete isolation and characterization of silybins and isosilybins from milk thistle (Silybum marianum). Organic & Biomolecular Chemistry 2003, 1, 1684-1689.; Smith, W. A.; Lauren, D. R.; Burgess, E. J.; Nigel, B. P.; Martin, R. J., A Silychristin Isomer and Variation of Falavonolignan Levels in Milk Thistle (Silybum marianum) Fruits Planta Medica 2005, 71, 877-880).

Weitere bekannte Nebenbestandteile sind 2,3-Dehydrosilybin, Desoxysilydianin (Silymonin), Silandrin, Silybinom, Silyhermin und Neosilyhermin.

Im Stand der Technik werden zumeist die gesamten Früchte der Mariendistel zur Extraktherstellung verwendet.

Bekannt ist zudem ein Mariendistelfrüchtetrockenextrakt (Extr. Cardui mariae fruct. Siccum), welcher u.a. mit dem Extraktionsmittel Ethylacetat aus der Pflanzendroge erhalten wird und nach der geltenden Ph. Eur. standardisiert ist (Monographie).

Die deklarierten Anforderungen an einen Trockenextrakt belaufen sich auf einen Gehalt von vorzugsweise 30 - 65 Gew. % Silymarin (andere Gehaltsspannen sind möglich), wobei der Anteil an Silymarin folgendermaßen zusammengesetzt ist:
40-65 Gew.% (relativer Anteil am Gesamtsilymarin): Silybin A und B (Diastereomerengemisch, C₂₅H₂₂OH₁₀ MG 482,4 (CAS 22888-70-6)) und
10-20 Gew.% (relativer Anteil am Gesamtsilymarin): Isosilybin A und B (Diastereomerengemisch , C₂₅H₂₂OH₁₀ MG 482,4 (CAS 72581-71-6)) und
20-45 Gew.% (relativer Anteil am Gesamtsilymarin): Silydanin und Silychristin (C₂₅H₂₂OH₁₀ MG 482,4).

Diese Flavonolignane sind in Wasser unlöslich bis schwer löslich (Löslichkeit von reinem Silymarin beträgt bei pH 6,9 ca. 0,08 mg/ml) und zeigen eine geringe Bioverfügbarkeit.

Zur Herstellung eines Extraktes wird das Rohmaterial (hier: Pflanzendroge) im Stand der Technik in hochaufwändiger Weise entfettet, extrahiert, filtriert, (auf)konzentriert und gereinigt.

Üblicherweise wird nach kontinuierlicher Extraktion mit Ethylacetat / Ethanol / Aceton / Methanol (ggfs. wässrig) oder wässrigen Mischungen mit den vorher genannten Lösungsmitteln eine Filtration durchgeführt mit anschließender (Auf)Konzentration. Nachfolgend erfolgt zumeist eine Aufreinigung mit Ethanol und Hexan (weitere Entfettung), so dass der oben genannte Gehalt an Silymarin erhalten werden kann.

Daher besteht die Aufgabe einen Mariendistelextrakt enthaltend Silymarin in einem verbesserten Verfahren bereit zu stellen. Insbesondere soll der native Charakter beibehalten werden.

Überraschender Weise wurde gefunden, dass Silymarin in den Schalen der Mariendistelfrüchte hochangereichert ist, auch nach erster mechanischer Trennung der Schale vom Kern. Zwar beschäftigt sich CN102219781 A mit der Herstellung eines Extraktes aus Früchteschalen, jedoch ohne Angabe einer geeigneten Extraktion und eines Solvens als auch ohne Produktspezifikation.

Im Rahmen der vorliegenden Erfindung wird unter "Silymarin" ein Stoffgemisch bezeichnet, welches (mindestens) die vier Substanzen Silybin, Silydianin, Silychristin und Isosilybin in unterschiedlichen Konzentrationen enthält. Dabei ist es unwesentlich, in welchen Verhältnissen diese Stoffe zueinander vorliegen und ob weitere Substanzen in dem Gemisch vorhanden sind. Diese Variation kann von der Herkunft und vom Geno- und Phänotyp der eingesetzten Mariendistel (Pflanzendroge) abhängen. Bevorzugt ist jedoch, dass diese Stoffe den Anforderungen des Ph. Eur. oder DAB in der jeweils geltenden Fassung erfüllen. Dies ist erfindungsgemäß der Fall.

Das Silymarin muss durch geeignete Extraktionstechniken aus der Schale gewonnen werden. Durch die Einbettung des Silymarins in die holzige Matrix sind diese durch direkte Extraktion jedoch nur unzureichend zugänglich. Die vorliegende Erfindung stellt sich ebenfalls die Aufgabe dieses technische Problem zu lösen.

Daher betrifft die Erfindung ein Verfahren zur Herstellung eines Mariendistelextraktes aus Mariendistelfrüchteschalen enthaltend Silymarin mittels Extraktion (nachstehend erfindungsgemäßes Verfahren).

Der Begriff "(Mariendistel)Früchteschalen" ist mit dem Begriff "Samenschalen" vor- und nachstehend synonym zu lesen. Im Rahmen dieser Erfindung wird unter dem Begriff "(Mariendistel)Früchteschalen" die Schalen der Früchte als auch der Samen der Mariendistel, einschließlich der Achänen, verstanden, welche vom Kern befreit sind (Im Folgenden kurz: "Schale").

Besonders vorteilhaft ist, dass der Gehalt von Silymarin am Gesamtmaterial der Schalen gegenüber den herkömmlichen Verfahren erhöht ist, da im Kern der Früchte bzw. Samen sehr wenig Silymarin enthalten ist. Zudem ist eine aufwändige Entfettung der Mariendistelfrüchteschalen wie z.B. bei einem herkömmlichen Mariendistelextrakt nicht erforderlich. Daher können mittels des erfindungsgemäßen Verfahrens höhere Raum-Zeit-Ausbeuten bzw. Extraktausbeuten erreicht werden. Zudem zeigen Vergleichsversuche, dass eine verbesserte Qualität und Reinheit der erhaltenen erfindungsgemäßen Mariendistelfrüchteschalenextrakte erreicht werden kann (siehe Beispiele und Abbildungen).

Die Erfindung betrifft daher ein Verfahren zur Herstellung eines Mariendistelextrakts aus Früchteschalen, mit den folgenden Schritten
a) Schälen der Früchte der Mariendistel, und Abtrennen der erhaltenen Schalen,
b) Entfetten der Schalen aus a.), und
c) Quellen der Schalen in Wasser,
d) Extraktion der feuchten Schalen aus c.) in Aceton (Dipolmoment: 10,0 x 10E-30 Cm) oder einem Lösungsmittel mit einem Dipolmoment von 8 - 12 x 10E-30 Cm,
e) Optionale Flüssigphasenextraktion des Überstandes aus d.) mit Heptan (Dipolmoment: 0,0 x 10E-30 Cm) oder mit einem Lösungsmittel oder Gas mit einem Dipolmoment von 0,0 x 10E-30 Cm,
f) Trocknen der Überstände aus d.) und / oder e.).

Das erfindungsgemäße Verfahren erlaubt die Herstellung eines Mariendistelextrakts aus Früchteschalen.

Daher betrifft die Erfindung weiterhin einen Mariendistelextrakt, der aus Schalen der Mariendistelfrüchte gemäß dem erfindungsgemäßen Verfahren erhalten werden kann oder erhältlich ist, insbesondere ein Trockenextrakt.

In weiteren bevorzugten Ausführungsformen umfasst die Erfindung die folgenden Schritte:
Weiterhin ist bevorzugt, dass die Schalen gemäß Schritt a.) mechanisch zerkleinert werden, insbesondere können diese in Form eines Mehls oder Pulvers vorliegen. In einer weiteren bevorzugten Ausführungsform können die Schalen zum leichteren Aufschluss mit Enzymen vor- oder nachbehandelt werden, insbesondere solche wie Cellulasen (Enzymklasse 3.2.1.4) oder Glucosidasen (Enzymklasse 3.2.1.21).

Desweiteren können die Mariendistelfrüchteschalen mit Säuren oder Laugen zum leichteren Aufschluss vor- oder nachbehandelt werden. Geeignete Säuren sind z.B. Mineralsäuren, wie Schwefelsäure oder Salzsäure. Geeignete Lauge ist z.B. Natronlauge.

Weiterhin können die schalentragenden Mariendistelfrüchte zur besseren Schalung und Aufschluss erhitzt werden.

Geeignete Lösungsmittel zum Entfetten der Schalen gemäß Schritt b.) sind insbesondere solche unpolaren Lösungsmittel mit einem Dipolmoment von 0,0 x 10E-30 Cm, wie Hexan, Heptan, Cyclohexan, Kohlendioxid, Kohlenstoffdisulfid, wobei Heptan und Kohlendioxid bevorzugt ist. Das anzuwendende Extraktionsverfahren oder Waschen ist dem Fachmann einschlägig bekannt und gängig. Bevorzugt ist, dass die Extraktion bzw. das Waschen mindestens eine Stunde beträgt und bei Temperaturen von 30 bis 80 Grad C, insbesondere 35 bis 70 Grad C durchgeführt wird. Fakultativ kann ein Vakuum angelegt werden.

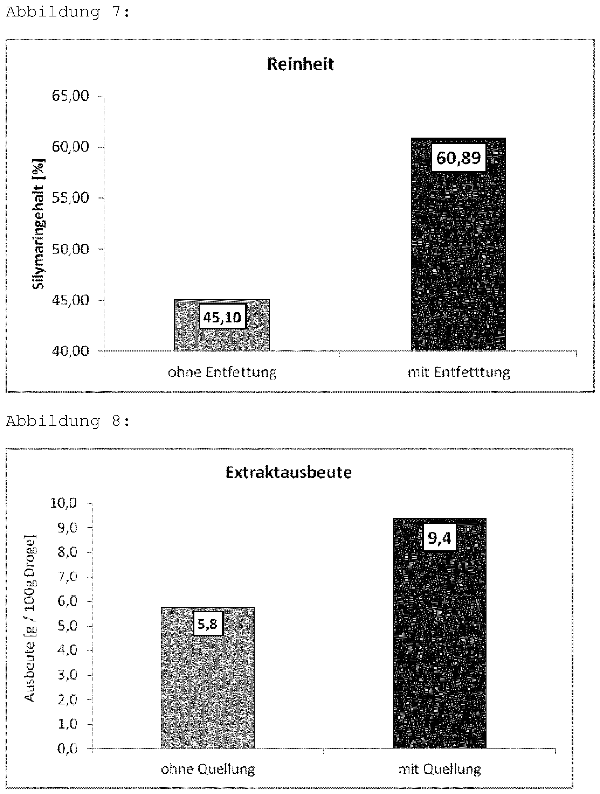

Weiterhin kann das Entfetten gemäß Schritt b.) vorteilhaft mittels über- und unterkritischen Gasen erfolgen, insbesondere im Rahmen der so genannten Superfluiden (SF) in einer SF-Extraktion, wie Kohlendioxid u.a. SF-Extraktionsverfahren sind für Pflanzenextrakte dem Fachmann einschlägig bekannt und gängig. So werden zum Beispiel ätherische Öle bei Temperaturen um 40°C und Drücken um 85 bar technisch aus Pflanzen(drogen)material gewonnen, wobei diese separat von Wachsen und fetten Ölen anfallen, die bei gleicher Temperatur erst ab Drücken über 280 bar extrahiert werden (siehe z.B.: Ernesto Reverchon und Iolanda De Marco: Supercritical fluid extraction and fractionation of natural matter (Review), Journal of Supercritical Fluids 38 (2006) 146 - 166). Vorteilhaft erfolgt ebenfalls eine Steigerung der Reinheit des Mariendistelextrakts aus Früchteschalen bei Entfettung der Schalen vorzugsweise mit Heptan oder superkritischem CO₂ (Schritt b.)), wobei der Silymaringehalt erhöht ist (siehe Abbildung 7). Die Reinheit des erhaltenen Mariendistelextrakts aus Früchteschalen gemäß dem erfindungsgemäßen Verfahren steigt um 35%, ausgewiesen am Silymaringehalt.

Nach dem Entfetten wird das entsprechende Lösungsmittel oder Gas von der Vorlage (Früchteschalen) entfernt.

In einer weiteren bevorzugten Ausführungsform werden die entfetteten Schalen mit Wasser gemäß Schritt c.) behandelt, dies kann mittels Extraktion oder Waschen ggfs. unter Rühren erfolgen. Erfindungsgemäß erfolgt ein gewünschtes Aufquellen der Früchteschalen. Dies ist von besonderem Vorteil, da mittels Quellen im Folgeschritt das Silymarin besonders effizient aus den Früchteschalen gelöst werden kann. Die Behandlung der Schalen mit (Heiß-)Wasser unter Quellung in Schritt c.) beeinflusst eine nachfolgende Extraktion äußerst positiv. Die Ausbeute des erhaltenen Extrakts nach Extraktion steigt um 63% (siehe Abbildung 8) berechnet nach der Ausgangsmenge der Droge.

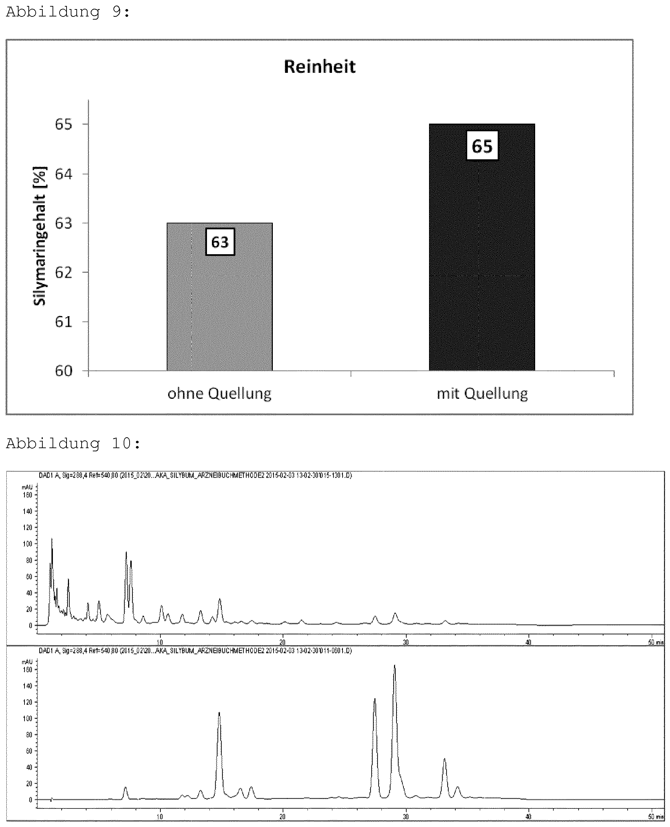

Vorteilhaft erfolgt ebenfalls eine Steigerung der Reinheit des Mariendistelextrakts aus Früchteschalen bei Behandlung der Schalen mit (Heiß-)Wasser (= Quellung, Schritt c.)), wobei der Silymaringehalt signifikant immer erhöht ist (siehe Abbildung 9).

Bevorzugt ist, dass die Extraktion bzw. das Waschen mindestens eine Stunde beträgt und bei Temperaturen von 50 bis 90 Grad C, insbesondere 70 Grad C durchgeführt wird. Fakultativ kann ein Vakuum angelegt werden.

Das Aufquellen bewirkt ein Aufschließen der Schalen und zusätzliches Waschen unter Entzug von hydrophilen Verunreinigungen (Abbildung 10) unter Anreicherung von Silymarin.

Nach dem Quellen wird das Wasser inkl. Verunreinigungen von der Vorlage (Früchteschalen) entfernt, wobei feuchte Früchteschalen zurückbleiben.

Anschließend erfolgt gemäß Schritt d.) eine Extraktion der feuchten Schalen in Aceton (Dipolmoment: 10,0 x 10E-30 Cm) oder einem Lösungsmittel mit einem Dipolmoment von 8 - 12 x 10E-30 Cm (z.B. Essigsäureanhydrid, Acetonitril, Butanon). Bevorzugt ist, dass die Extraktion bzw. Waschen mindestens 3 Stunden beträgt und bei Temperaturen von 20 bis 30 Grad C, insbesondere 25 Grad C durchgeführt wird. Fakultativ kann ein Vakuum angelegt werden.

Der erhaltene Überstand wird abgetrennt, ggfs. aufkonzentriert, und getrocknet.

Der gemäß Schritt d.) erhaltene Überstand kann optional mittels einer Flüssigphasenextraktion mit Heptan (Dipolmoment: 0,0 x 10E-30 Cm) oder mit einem Lösungsmittel mit einem Dipolmoment von 0,0 x 10E-30 Cm (z.B. Hexan, Heptan, Cyclohexan, Kohlendioxid, Kohlenstoffdisulfid) oder über- und unterkritischen Gasen, insbesondere Kohlendioxid, erneut entfettet werden. Diese Extraktion kann mittels einem oder mehreren Lösungsmitteln erfolgen.

Insbesondere kann das Entfetten vorteilhaft mittels über- und unterkritischen Gasen erfolgen, insbesondere im Rahmen der so genannten Superfluiden (SF) in einer SF-Extraktion, wie Kohlendioxid u.a. SF-Extraktionsverfahren sind für Pflanzenextrakte dem Fachmann einschlägig bekannt und gängig (Ernesto Reverchon und Iolanda De Marco (supra)).

Nach dem Entfetten wird das entsprechende Lösungsmittel oder Gas von der vorgelegten Flüssigphase entfernt.

Der erhaltene Überstand wird abgetrennt, ggfs. aufkonzentriert, und getrocknet.

Daher betrifft die Erfindung ebenfalls einen Trockenextrakt erhältlich nach dem erfindungsgemäßen Verfahren enthaltend einen Silymaringehalt von mehr als 65%, insbesondere mehr als 67,5%, insbesondere mehr als 70%, insbesondere mehr als 75%.

Der erfindungsgemäße Trockenextrakt weist eine gelbliche bis beige Farbe auf und eine max. Feuchtigkeit von 5%, vorzugsweise 2-3 %. Insbesondere der Verfahrensschritt c.) bewirkt, dass die Farbe des erfindungsgemäßen Trockenextrakts immer heller ist, als wenn kein Schritt c) erfolgt. Dies liegt an der erhöhten Reinheit und Anreicherung von Silymarin, insbesondere des weißen Silybins.

Trockenextrakte gemäß Schritt e.) können in üblicher Weise hergestellt werden, wobei mit Hilfe eines Lösungsmittels oder Lösungsmittelgemisches extrahiert, beispielsweise mittels (Bewegungs-)Mazeration oder Perkolation - welche erfindungsgemäß mitumfasst sind - und nach Abtrennung des Extraktionsrückstandes, der erhaltene Extrakt zur Trockne eingeengt wird.

Herkömmliche Trocknungsverfahren sind erfindungsgemäß ebenfalls umfasst und schließen die Wirbelschichttrocknung oder die Einengung zu einem Dick- oder Spissumextrakt und anschließende Vakuumbandtrocknung oder Hordentrocknung dieses Spissumextraktes ein.

Ebenfalls können klassische Verfahren zur Herstellung von erfindungsgemäßen Trockenextrakten berücksichtigt werden; wobei nach anschließender Destillation der Lösungsmittel ein so genannter Spissumextrakt (zähflüssiger Extrakt) erhalten wird, dem häufig Hilfsstoffe und/oder Zusatzstoffe wie z.B. Lactose, Polyvinylpyrrolidon, Saccharose, Siliciumdioxid usw. zugesetzt werden. Diese feuchte, zähe Masse wird dann in Hordenschränken oder Trocknern zur gewünschten Trockenextraktzubereitung gebracht.

Das erfindungsgemäße Verfahren erlaubt die Bereitstellung eines neuen Mariendistelextrakts aus Früchteschalen, wobei immer ein gesteigerter Gehalt an Silymarin erhalten wird, welches insbesondere auf Schritt c.) beruht. Das erfindungsgemäße Verfahren erlaubt sicher die maximale Anreicherung von Silymarin gegenüber dem Stand der Technik. Dies führt zudem in überraschender Weise zu einer verbesserten Freisetzung des Silymarins, wie Dissolutionstests in den Beispielen zeigen.

Ferner betrifft die Erfindung ebenfalls einen Mariendistelextrakt aus Früchteschalen und ein entsprechendes Arzneimittel oder Nahrungsergänzungsmittel und dessen Verwendung oder Anwendung zur Prophylaxe und Behandlung von Lebererkrankungen, Krebs- und Tumorerkrankungen, Leber- und Gallendysfunktionen, insbesondere von toxischen Leberschäden, insbesondere infolge einer Fettleber oder Alkoholmissbrauch, Hepatosen wie Pilzvergiftungen, akutes Leberversagen, Lebernekrose, Leberdystrophie, Leberzirrhose, Leberfibrose, Leberentzündung, Lebervergrößerung und Leberverfettung, Leberinsuffizienz, Hepatitis, insbesondere Hepatitis C, Darmkrebs, Leberkrebs, Hirntumor.

Ferner betrifft die Erfindung eine pharmazeutische Formulierung enthaltend einen erfindungsgemäßen Mariendistelextrakt aus Früchteschalen.

Die erfindungsgemäßen Mariendistelfrüchteschalenextrakte können in Form von pharmazeutischen Zubereitungen in Dosierungseinheiten zubereitet werden. Dies bedeutet, dass die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben, einem Drittel oder einem Viertel einer Tagesdosis entspricht. Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Formulierungen seien Tabletten, Dragees, Kapseln, Pillen, Granulate, Suppositorien, Lösungen, Saft, Suspensionen und Emulsionen genannt. Tabletten, Dragees, Kapseln, Pillen und Granulate können den Mariendistelfrüchteschalenextrakt neben den üblichen Trägerstoffen enthalten, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den Mariendistelfrüchteschalenextrakt nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der Mariendistelfrüchteschalenextrakt kann gegebenenfalls mit einem oder mehreren der oben angegebenen Trägerstoffe auch in mikroverkapselter Form vorliegen.

Suppositorien können neben dem Mariendistelfrüchteschalenextrakt die üblichen wasserlöslichen oder wasserunlöslichen Trägerstoffe enthalten, z.B. Polyethylenglykole, Fette, z.B. Kakaofett und höhere Ester (z.B. C14-Alkohol mit C16-Fettsäure) oder Gemische dieser Stoffe.

Lösungen und Emulsionen können neben dem Mariendistelfrüchteschalenextrakt die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3- Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Suspensionen können neben dem Mariendistelfrüchteschalenextrakt die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten. Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die Erfindung betrifft des Weiteren eine diätetische Zubereitung enthaltend eine physiologisch wirksame Dosis an erfindungsgemäßem Mariendistelextrakt aus Früchteschalen sowie einen physiologisch verträglichen Träger. Bei diätetischen Zubereitungen im Sinne der vorliegenden Erfindung handelt es sich um Zusammensetzungen, welche neben dem erfindungsgemäßen Mariendistelextrakt aus Früchteschalen ein Nahrungs- oder Lebensmittel (siehe z.B. nicht abschließend EU-Richtlinie 2002/46/EG vom 10. Juni 2002) umfassen, ggfs. enthaltend Hilfs- und Zusatzstoffe.

### Beispiele und Abbildungen:

Diese Beispiele dienen ausschließlich zur Erläuterung der Erfindung, ohne die Erfindung auf diese Beispiele zu beschränken.

### Beispiel 1:

### 1. Extraktion von Silymarin

### 1.1 Extraktion von Mariendistelfrüchten nach Arzneibuch Ph. Eur. 6.0/1860

Die Extraktion von Silybi mariani fructus nach Arzneibuch (Ph. Eur. 6.0/1860) ergibt einen deutlich gelb-gefärbten Extrakt, der die gleichen Hauptkomponenten wie der kommerziell erhältliche Mariendistelfrüchtetrockenextrakt enthält. Lediglich im vorderen Bereich des Chromatogramms sind neue Signale bei 22,5; 33,8 und 35,7 min sowie zahlreiche Minorkomponenten zwischen 0 und 20 min zu erkennen.

### 1.2 Untersuchung von Schalen und Kernen der Mariendistelfrüchte

### 1.2.1 Schälen der Mariendistelfrüchte

5,35 g Früchte wurden mechanisch gespalten und die Schalen von den Kernen getrennt. Dabei ergab sich ein recht ausgewogenes Verhältnis von 2,59 g Schalen (48 %) zu 2,76 g Kernen (52 %). Es wurden größere Mengen der jeweiligen Fraktionen bereitgestellt.

Es konnten aus 1 kg Mariendistelfrüchten 281 g Schalen und 341 g Kerne erhalten werden. Der Rest des Materials teilt sich in vier Mischfraktionen auf, die nicht weiter aufgereinigt werden können (5 g Leichtgut, 10 g Leichtkorn, 105 g Mischfraktion (ganz) und 255 g Mischfraktion (Mehl)).

### 1.2.2 Extraktion und Analytik von Schalen und Kernen

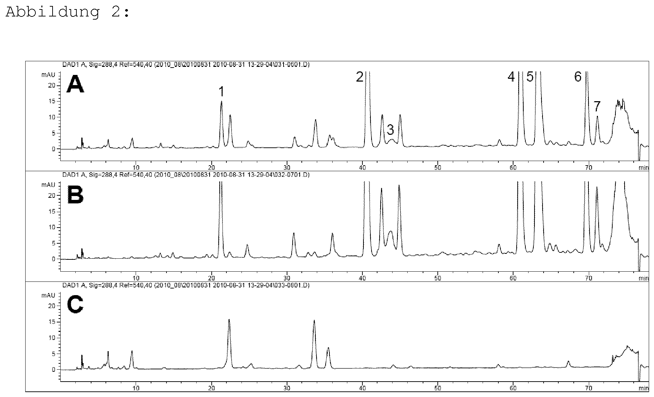

Für eine Bewertung des Inhaltsstoffprofils der Schalen und Kerne der Mariendistelfrüchte wurden die Proben pulverisiert und gemäß Arzneibuch (Ph. Eur. 6.0/1860) aufgearbeitet und nach Trocknen in 75 % Methanol aufgenommen wurde. In Abbildung 2 sind die erhaltenen HPLC-Chromatogramme dargestellt.

Erwartungsgemäß zeigt der Extrakt aus der Gesamtdroge (Abbildung 2A) ein Inhaltsstoffspektrum, dass der Summe der Extrakte aus Schalen (2B) und Kernen (2C) entspricht. Bemerkenswert ist hierbei jedoch, dass die Zusammensetzung von Schalen und Kernen grundsätzlich verschieden ist und nur in den Schalen Silymarin gefunden werden kann.

### Beispiel 2:

### 2. Vergleichende Freisetzungsversuche gemäß PhEur 1997, 2.9.3 (Dissolution-Test)

Der erfindungsgemäße Mariendistelextrakt aus Früchteschalen wird mit einem kommerziell erhältlichen Finzelberg-Extrakt aus gesamten Früchten verglichen.

### Versuchsparameter:

### Blattrührer-Apparatur (Auflösungsprüfgerät der Sotax AG AT, 7smart)

Dissolutionmedium: Pufferlösung pH 7,5 (0,02 M) R mit Zusatz von 5,0 % (m/V) Polysorbat 80 (Tween)
Mediumtemperatur: 37 Grad C
Umdrehungen pro Minute: 120
Probenentnahme: 15 ml (automatsch), solvent addition mit Entnahmezeitpunkte 0, 10, 20, 30, 45, 60, 90 und 120 Minuten
Feststoffabtrennung: automatische Filtration mit Filter GF/D, Fa. Whatman

### 3 Prüflinge:

| **Mat.-/EE-Nr.** | **Ch.-Bez.** | **Bezeichnung** | **Lieferant** |
|---|---|---|---|
| EE 0481 | 730279-3 | Cardui mariae | Bionorica-TE |
| EE 0481 | 730280-2 | Cardui mariae | Bionorica-TE |
| 2047301 | 063716 | Cardui mariae fruct. sicc. | Finzelberg |

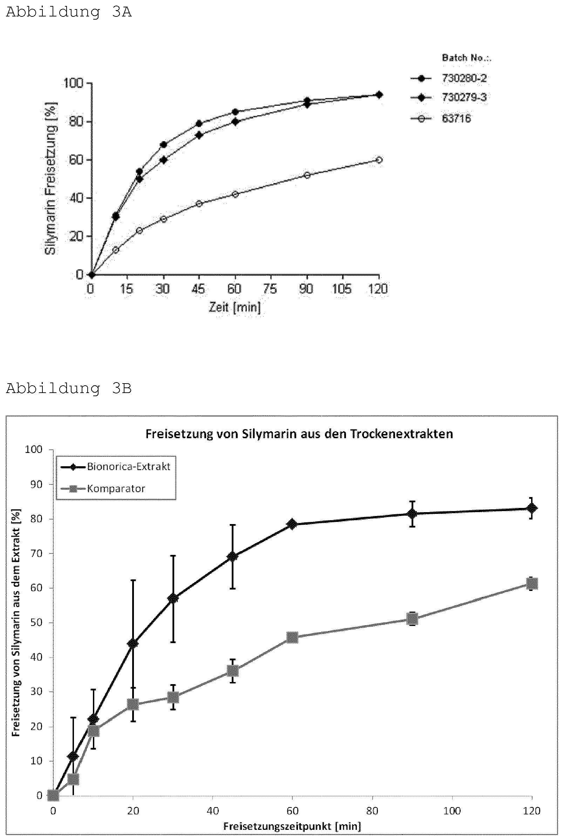

Die Ergebnisse sind in Abbildung 3A dargelegt. Die Mariendistelextrakte aus Früchteschalen 730279-3 und 730280-2 verhalten sich gegenüber dem eingesetzten Finzelberg Mariendistelextrakt aus Gesamtfrüchten 063716 in der Freisetzung (Dissolution) überlegen.

Ein weiterer entsprechender Dissolutionstest ist in Abbildung 3B ausgewiesen, wobei ein Mariendistelextrakt aus Früchteschalen gegenüber einem eingesetzten Mariendistelextrakt aus Gesamtfrüchten ("Komparator") (jeweils 3 Prüflinge) zum Endzeitpunkt (120 min.) eine um 22% (absolut), beziehungsweise 36% (relativ) gesteigerte Freisetzung von Silymarin aus einem Trockenextrakt zeigt.

### Beispiel 3:

### 3.1 Analytischer Vergleich zwischen

### Mariendisteltrockenextrakten aus Silybum Schalen und Gesamtdroge (Silybi mariani fructus)

### Silymaringehalte:

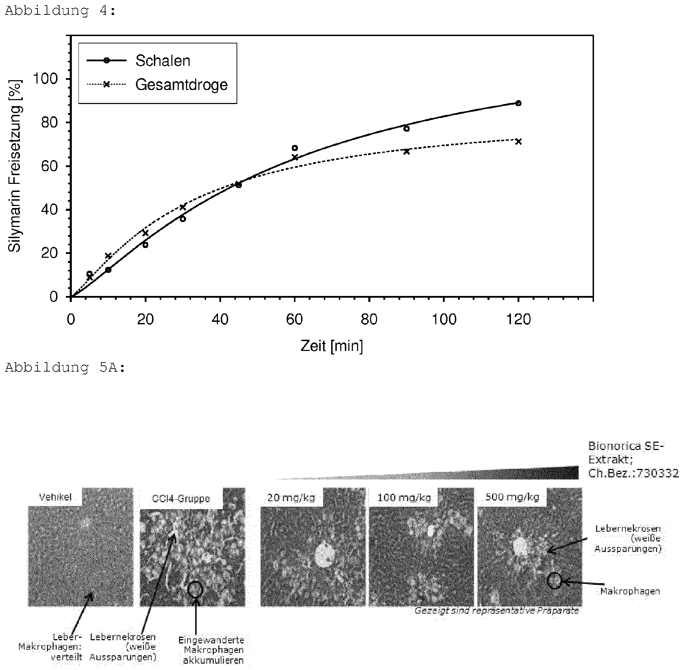

Mariendisteltrockenextrakte aus Schalen weisen 3-4 % höhere Silymaringehalte als Extrakte aus Gesamtfrüchten auf. Die Extrakte aus den Kernen (geschälte Früchte) sind frei von Silymarin (Spuren aus den anhaftenden Schalenresten nachweisbar). Die Ergebnisse sind in Abbildung 4 dargestellt.

Aus den Freisetzungsexperimenten gemäß Abbildung 4 geht hervor, dass der Mariendisteltrockenextrakt aus Schalen eine bessere Freisetzung von Silymarin aufweist.

### Beispiel 4:

### 4. Pharmakodynamische Untersuchung des Mariendistelextrakte aus Früchteschalen bezüglich der Protektion bei CCl₄-induziertem Leberschaden in vivo

Prüfmuster: Ch.Bez.:730332

### 4.1 Experiment: Tetrachlorkohlenstoff (CCl₄)-induzierte akute Hepatotoxizität

Spezies: Sprague Dawley CD® Ratten, Männchen
Dosierungen: 3 (20, 100, 500 mg/kg per os); Gabe 3h vor der CCl₄-Behandlung
Anzahl: N=22, (4 Tiere Vehikel Kontrolle, 6 CCl₄-Kontrolltiere, je 4 Tiere in der Behandlungsgruppe)
Zeitpunkt der Organentnahme: 24 h

### Analyseparameter:

Parameter: Gewebeschädigung
Methode: Histologie
Parameter: Expression von Entzündungsmediatoren
Methode: quantitative PCR

### Ergebnisse:

Der Effekt von Mariendistelextrakt aus Früchteschalen auf CCl₄-induzierten Leberschaden (in vivo) ist in Abbildung 5a dargestellt.

Die Behandlung mit dem Mariendistelextrakt aus Früchteschalen bewirkt eine deutliche Verringerung der Lebernekrosen und eine Reduktion der Einwanderung an Immunzellen. Folglich schützt der Mariendistelextrakt aus Früchteschalen vor Tetrachlorkohlenstoff (CCl₄)-induziertem Leberschaden.

### 4.2 Effekt von Mariendistelextrakt aus Früchteschalen auf die Expression von inflammatorischen Zytokinen in der Leber (in vivo)

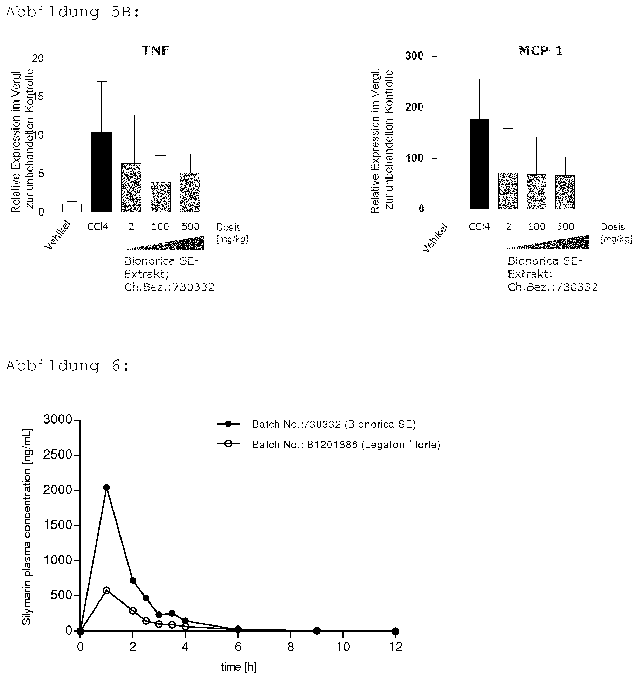

Die Ergebnisse sind in Abbildung 5b dargestellt.

Der Mariendistelextrakt aus Früchteschalen zeigt eine tendenzielle Reduktion der Expression der proinflammatorischen Zytokine TNF und MCP-1.

Der Mariendistelextrakt aus Früchteschalen beugt der Tetrachlorkohlenstoff (CCl₄)-induzierten Entzündung vor.

### Beispiel 5: Messung der Silymarin Konzentration im Plasma von CD Mäusen im Tierexperiment

### a.) Prüfmuster:

Bionorica SE: Ch.Bez.:730332 (+6% SiO₂) (erfindungsgemäßer Mariendistelextrakt aus Früchteschalen),
Handelspräparat Legalon forte®: Ch.Bez.: B1201886 (+ ≈ 49% Hilfsstoffe) (Mariendistelfrüchteextrakt).

### b.) Tierexperiment:

Spezies: CD® Ratten m/w (1:1),
Dosierung: einmalig per os 500 mg/kg bezogen auf den
jeweiligen Extrakt,
Anzahl: N=90, d.h. 6 Werte pro Blutabnahmezeitpunkt,
Abnahmezeitraum: 0- 12 h.

### c.) Bioanalytik:

Methode: HPLC-MS/MS (validiert),
Matrix: Plasma Ratte,
Bioanalyt: Silybin, Isosilybin (Silymarin),
LLOQ (untere Quantifizierungsgrenze): 12.5 ng/mL.

Die Ergebnisse sind in Abbildung 6 dargestellt und Gegenstand nachstehender Tabelle 5.1.

***geometrischer Mittelwert **Median**

| PK-Parameter Silymarin | Dosis mg/kg | AUC* [h*ng/mL] | Cₘₐₓ* [ng/mL] | tₘₐₓ** [h] |
|---|---|---|---|---|
| Ch.Bez.: 730332 (+6% SiO₂) | 500 | 3099 | 1876 | 1.00 |
| Ch.Bez.: B1201886 (+ ≈ 49% Hilfsstoffe) | 500 | 1081 | 553 | 1.00 |

Der erfindungsgemäße Mariendistelextrakt aus Früchteschalen zeigt gegenüber einem Mariendistelfrüchteextrakt eine 2,9 fache Erhöhung des AUC als auch eine 3,4 fache Erhöhung von Cₘₐₓ (siehe auch Abbildung 6).

### Beschreibung der Abbildungen:

Abbildung 1 zeigt die wesentlichen Flavonolignane.
Abbildung 2 zeigt HPLC-UV Chrommatogramme (λ0288 nm) von Silybi mariani fructus (Extraktion nach Ph. Eur. 6.0/1860); pulverisiert Früchte (A), pulverisierte Schalen (B), pulverisierte Kerne (C) mit Zuweisung für Taxifolin (1), Silychristin (2), Silydianin (3), Silybin A (4), Silybin B (5), Isosilybin A (6), Isosilybin B (7).
Abbildungen 3A und 3B zeigen jeweils einen Dissolution-Test gemäß Beispiel 2.
Abbildung 4 zeigt Dissolutionexperimente von nativen Trockenextrakten aus Silybum marianum (aus Schalen durchgezogene Linie, aus Gesamtdroge gepunktete Linie), Mittelwertkurven aus je 3 Bestimmungen.
Abbildung 5A zeigt den Effekt von Mariendistelextrakt aus Früchteschalen auf CCl₄-induzierten Leberschaden (in vivo).
Abbildung 5B zeigt den Effekt von Mariendistelextrakt aus Früchteschalen auf die Expression von inflammatorischen Zytokinen in der Leber im Modell des CCl₄-induzierten Leberschadens (in vivo).
Abbildung 6 zeigt Vergleichsversuche zur Silymarin Konzentration im Plasma von CD Mäusen zwischen dem erfindungsgemäßen Mariendistelextrakt aus Früchteschalen mit handelsüblichem Legalon forte® (Mariendistelfrüchteextrakt).
Abbildung 7: Steigerung der Reinheit des Mariendistelextrakts aus Früchteschalen bei Behandlung der Schalen mit oder ohne Entfettung, vorzugsweise Heptan oder superkritischem CO₂ (Schritt b.)) ausgewiesen am Silymaringehalt.
Abbildung 8: Vergleich der Extraktausbeuten mit und ohne Behandlung der Schalen mit (Heiß-)Wasser (= Quellung, Schritt c.)), berechnet nach der Ausgangsmenge der Droge.
Abbildung 9: Steigerung der Reinheit des Extrakts bei Behandlung der Schalen mit (Heiß-)Wasser (= Quellung, Schritt c.)) ausgewiesen am Silymaringehalt.
Abbildung 10 zeigt den Entzug von hydrophilen Verunreinigungen / Substanzen aus den Schalen beim Quellen (oben, Waschwasser der Quellung) und unten, dem erhaltenen erfindungsgemäßen Trockenextrakt (Silybin a und B sind deutlich sichtbar). Dies beweist die hohe Reinheit und wie effizient diese hydrophilen Verunreinigungen durch den Schritt c) entfernt werden.

## Patentansprüche

1. Verfahren zur Herstellung eines Mariendistelextrakts aus Früchteschalen, mit den folgenden Schritten:
a.) Schälen der Früchte der Mariendistel, und Abtrennen der erhaltenen Schalen,
b.) Entfetten der Schalen aus a.), und
c.) Quellen der Schalen in Wasser,
d.) Extraktion der feuchten Schalen aus c.) in Aceton (Dipolmoment: 10,0 x 10E-30 Cm) oder einem Lösungsmittel mit einem Dipolmoment von 8 - 12 x 10E-30 Cm,
e.) Trocknen des Überstandes.

2. Verfahren zur Herstellung eines Mariendistelextrakts aus Früchteschalen nach Anspruch 1, wobei eine Flüssigphasenextraktion des Überstandes aus d.) mit Heptan (Dipolmoment: 0,0 x 10E-30 Cm) oder mit einem Lösungsmittel mit einem Dipolmoment von 0,0 x 10E-30 Cm oder über- und unterkritischen Gasen, insbesondere Kohlendioxid, und anschließend gemäß e.) ein Trocknen des Überstandes erfolgt.

3. Verfahren zur Herstellung eines Mariendistelextrakts aus Früchteschalen nach einem der vorstehenden Ansprüche, **dadurch gekennzeichnet, dass** in Schritt a.) die Schalen wahlweise i.) erhitzt und/oder ii.) mit einem Enzym behandelt und/oder iii.) mit Säure oder Lauge behandelt werden.

4. Verfahren zur Herstellung eines Mariendistelextrakts aus Früchteschalen nach einem der vorstehenden Ansprüche, wobei ein Entfetten der Früchteschalen gemäß Schritt b.) mittels Extraktion oder Waschen mit einem oder mehreren Lösungsmitteln, insbesondere Heptan (Dipolmoment: 0,0 x 10E-30 Cm) oder mit einem Lösungsmittel mit einem Dipolmoment von 0,0 x 10E-30 Cm, oder über- und unterkritischen Gasen, insbesondere Kohlendioxid, erfolgt.

5. Mariendistelextrakt aus Früchteschalen erhältlich nach einem Verfahren nach einem der Ansprüche 1 bis 4, insbesondere ein Trockenextrakt davon.

6. Trockenextrakt erhältlich aus einem Mariendistelextrakt aus Früchteschalen nach Anspruch 5, enthaltend einen Silymaringehalt von mehr als 65 %, insbesondere mehr als 67,5 %, insbesondere mehr als 70 %, insbesondere mehr als 75 %.

7. Trockenextrakt erhältlich aus einem Mariendistelextrakt aus Früchteschalen nach Anspruch 5, wobei die Feuchtigkeit max. 5 % beträgt.

8. Arzneimittel oder Nahrungsergänzungsmittel enthaltend einen Mariendistelextrakt aus Früchteschalen nach einem der Ansprüche 5 bis 7.

9. Arzneimittel oder Nahrungsergänzungsmittel enthaltend einen Mariendistelextrakt aus Früchteschalen nach einem der Ansprüche 5 bis 7 zur Verwendung in der Prophylaxe und/oder Behandlung von Leber- und Gallendysfunktionen, insbesondere von toxischen Leberschäden, Hepatosen wie Pilzvergiftungen, akutes Leberversagen, Lebernekrose, Leberdystrophie, Leberzirrhose, Leberfibrose, Leberentzündung, Lebervergrößerung und Leberverfettung, Leberinsuffizienz, Hepatitis, insbesondere Hepatitis C.

10. Arzneimittel oder Nahrungsergänzungsmittel enthaltend einen Mariendistelextrakt aus Früchteschalen nach einem der Ansprüche 5 bis 7 zur Verwendung in der Prophylaxe und/oder Behandlung von Krebs- und Tumorerkrankungen, insbesondere Darmkrebs, Leberkrebs, Hirntumor.

11. Pharmazeutische oder diätetische Zubereitung enthaltend einen Mariendistelextrakt nach einem der Ansprüche 5 bis 7, ggfs. Hilfs- und Zusatzstoffe.

## Claims

1. A method for producing a milk thistle extract from fruit peels, said method comprising the following steps:
a.) peeling the fruit of milk thistle and separating the peels obtained,
b.) removing the fat from the peels from a.), and
c.) swelling the peels in water,
d.) extracting the moist peels from c.) in acetone (dipole moment: 10.0 x 10E-30 Cm) or a solvent with a dipole moment of 8 - 12 x 10E-30 Cm,
e.) drying the supernatant.

2. The method for producing a milk thistle extract from fruit peels according to claim 1, wherein a liquid phase extraction of the supernatant from d.) is performed with heptane (dipole moment: 0.0 x 10E-30 Cm) or with a solvent with a dipole moment of 0.0 x 10E-30 Cm or supercritical and subcritical gases, in particular carbon dioxide, and then a drying of the supernatant according to e.) is performed.

3. The method for producing a milk thistle extract from fruit peels according to either one of the preceding claims, **characterised in that** in step a.) the peels are optionally i.) heated and/or ii.) treated with an enzyme and/or iii.) are treated with acid or lye.

4. The method for producing a milk thistle extract from fruit peels according to any one of the preceding claims, wherein the fat is removed from the fruit peels in accordance with step b.) by means of extraction or washing with one or more solvents, in particular heptane (dipole moment: 0.0 x 10E-30 Cm) or with a solvent with a dipole moment of 0.0 x 10E-30 Cm, or supercritical and subcritical gases, in particular carbon dioxide.

5. A milk thistle extract from fruit peels obtainable by a method according to any one of claims 1 to 4, in particular a dry extract thereof.

6. A dry extract obtainable from a milk thistle extract from fruit peels according to claim 5, containing a silymarin content of more than 65%, in particular more than 67.5%, in particular more than 70%, in particular more than 75%.

7. A dry extract obtainable from a milk thistle extract from fruit peels according to claim 5, wherein the moisture is at most 5%.

8. A medicinal product or food supplement containing a milk thistle extract from fruit peels according to any one of claims 5 to 7.

9. A medicinal product or food supplement containing a milk thistle extract from fruit peels according to any one of claims 5 to 7 for use in the prophylaxis and/or treatment of liver and gall dysfunctions, in particular of toxic liver damage, hepatoses such as fungus poisoning, acute liver failure, liver necrosis, liver dystrophy, liver cirrhosis, liver fibrosis, inflammation of the liver, enlargement of the liver and fatty liver, liver insufficiency, hepatitis, in particular hepatitis C.

10. A medicinal product or food supplement containing a milk thistle extract from fruit peels according to any one of claims 5 to 7 for use in the prophylaxis and/or treatment of cancer and tumour disorders, in particular colon cancer, liver cancer, brain tumour.

11. A pharmaceutical or dietetic preparation containing a milk thistle extract according to any one of claims 5 to 7, and optionally excipients and additives.

## Revendications

1. Procédé de préparation d'un extrait de chardon-marie à partir des coques de fruits, avec les étapes suivantes :
a.) pelage des fruits de chardon-marie, et séparation des coques obtenues,
b.) huilage des coques provenant de a.), et
c.) gonflement des coques dans de l'eau,
d.) extraction des coques humides provenant de c.) dans de l'acétone (moment dipolaire : 10,0 x 10Ex-30 cm) ou dans un solvant avec un moment dipolaire de 8 - 12 x 10Ex-30 cm,
e.) séchage du résidu.

2. Procédé de préparation d'un extrait de chardon-marie à partir des coques de fruits selon la revendication 1, où une extraction en phase liquide du résidu provenant de d.) a lieu avec de l'heptane (moment dipolaire : 0,0 x 10Ex-30 cm) ou avec un solvant avec un moment dipolaire de 0,0 x 10Ex-30 cm ou avec des gaz supercritiques et sous-critiques, notamment du dioxyde de carbone, et ensuite un séchage du résidu a lieu selon e.).

3. Procédé de préparation d'un extrait de chardon-marie à partir des coques de fruits selon l'une des revendications précédentes, **caractérisé en ce que**, dans l'étape a.), les coques sont soit i.) chauffées, et/ou ii.) traitées avec une enzyme, et/ou iii.) traitées avec de l'acide ou une base.

4. Procédé de préparation d'un extrait de chardon-marie à partir des coques de fruits selon l'une des revendications précédentes, où un huilage des coques de fruits a lieu selon l'étape b.) au moyen d'une extraction ou d'un lavage avec un ou plusieurs solvants, notamment de l'heptane (moment dipolaire : 0,0 x 10Ex-30 cm) ou avec un solvant avec un moment dipolaire de 0,0 x 10Ex-30 cm, ou des gaz supercritiques ou sous-critiques, notamment du dioxyde de carbone.

5. Extrait de chardon-marie provenant des coques de fruits pouvant être obtenu d'après un procédé selon l'une des revendications 1 à 4, notamment un extrait sec de celui-ci.

6. Extrait sec pouvant être obtenu à partir d'un extrait de chardon-marie provenant des coques de fruits selon la revendication 5, contenant une teneur en silymarine supérieure à 65 %, notamment supérieure à 67,5 %, notamment supérieure à 70 %, notamment supérieure à 75 %.

7. Extrait sec pouvant être obtenu à partir d'un extrait de chardon-marie provenant des coques de fruits selon la revendication 5, où l'humidité est au maximum de 5 %.

8. Médicament ou complément alimentaire contenant un extrait de chardon-marie provenant des coques de fruits selon l'une des revendications 5 à 7.

9. Médicament ou complément alimentaire contenant un extrait de chardon-marie provenant des coques de fruits selon l'une des revendications 5 à 7 pour l'utilisation dans la prophylaxie et/ou le traitement de disfonctionnements du foie et de la vésicule, notamment d'endommagements toxiques du foie, d'hépatoses comme des empoisonnements par des champignons, de défaillance aigüe du foie, de nécrose du foie, de dystrophie du foie, de cirrhose du foie, de fibrose du foie, d'inflammation du foie, d'augmentation de la taille du foie et de stéatose du foie, d'insuffisance hépatique, d'hépatite, notamment d'hépatite C.

10. Médicament ou complément alimentaire contenant un extrait de chardon-marie provenant des coques de fruits selon l'une des revendications 5 à 7 pour une utilisation dans la prophylaxie et/ou le traitement de maladies cancéreuses et tumorales, notamment du cancer de l'intestin, du cancer du foie, d'une tumeur du cerveau.

11. Préparation pharmaceutique ou diététique contenant un extrait de chardon-marie selon l'une des revendications 5 à 7, éventuellement, des excipients et des additifs.
